# EUROPÄISCHE PATENTSCHRIFT

(11) **EP 3 668 563 B1**
(45) Veröffentlichungstag und Bekanntmachung des Hinweises auf die Patenterteilung: **03.11.2021**
(21) Anmeldenummer: 18740524.6
(22) Anmeldetag: 02.07.2018
(51) Int. Cl.: A61M 1/36, A61M 25/02

(54) **KATHETER FÜR DIE DIALYSE**
CATHETER FOR DIALYSIS
CATHÉTER POUR LA DIALYSE

(30) Priorität: 17.08.2017 DE 102017118820
(43) Veröffentlichungstag der Anmeldung: 24.06.2020
(73) Patentinhaber: Joline GmbH & Co. KG, 72379 Hechingen (DE)
(72) Erfinder: SEIDENBERGER, Dieter, 72379 Hechingen (DE)
(74) Vertreter: DREISS Patentanwälte PartG mbB
(86) Internationale Anmeldenummer: PCT/EP2018/067755
(87) Internationale Veröffentlichungsnummer: WO 2019/034320

(56) Entgegenhaltungen:
- GB-A- 2 422 553
- US-A1- 2004 199 122
- US-A1- 2010 016 801
- US-A1- 2014 228 810
- US-B1- 6 283 945

## Beschreibung

Die Erfindung betrifft einen Katheter für die Dialyse, insbesondere zur Langzeitanwendung, mit einem proximalen Ende zum Einführen in ein Blutgefäß, mit einem distalen Ende zur Entnahme von Blut und zur Einführung von gereinigtem Blut, und mit wenigstens einem einen Schlauchabschnitt umgebenden Fixiermittel zur Fixierung des Katheters am Patienten. Unter dem Begriff der Dialyse werden im Folgenden sämtliche Blutreinigungsverfahren verstanden, insbesondere die Hämodialyse, Hämofiltration, Hämodiafiltration, Peritonealdialyse, Hämoperfusion sowie Apherese-Verfahren.

Eingangs genannte Katheter sind aus dem Stand der Technik in vielfältiger Art und Weise bekannt. Insbesondere bei Langzeitanwendungen verbleiben diese Katheter mehrere Tage, mehrere Wochen oder mehrere Monate im Patienten.

Aus der DE 602 25 431 T2 und der GB 2 422 553 A sind solche Katheter bekannt, die ein Fixiermittel zur Fixierung des Katheters am Patienten in Form eines Nahtfadenrings aufweisen, die Fadenlöcher vorsieht, mittels welcher der Katheter an dem Patienten angenäht werden kann. Aus der US 6 283 945 B1 und US 2004 0 199 122 A1 sind Katheter mit Merkmalen des Oberbegriffs des Patentanspruchs 1 bekannt, die eine alternative Befestigungsmöglichkeit des Katheters am Patienten über Clips auf adhäsiven Kissen vorsehen. In US 2010 0 016 801 A1 und US 2014 0 228 810 A1 wird die Befestigung des Katheters mittels Haken unterhalb der Haut offenbart.

Nachteilig beim bekannten Stand der Technik ist, dass die Zentriermittel beim Bewegen des Katheters störend im Weg sind. Zudem kann es in der Langzeitanwendung vorteilhaft sein, ein Fixiermittel, das nahe an der Eintrittsstelle des Katheters in den Körper fixiert ist aus hygienischen Gründen ausgetauscht oder entfernt werden muss.

Der Erfindung liegt die Aufgabe zugrunde, einen Katheter der eingangs genannten Art vorzuschlagen, der auf einfache Art und Weise austauschbar ist.

Diese Aufgabe wird gelöst durch einen Katheter mit den Merkmalen des Patentanspruchs 1. Der Katheter sieht folglich vor, dass das Fixiermittel ein Aufnahmeteil und ein Öffnungsteil aufweist, wobei das Öffnungsteil gegenüber dem Aufnahmeteil aus einer Öffnungsstellung in der der Schlauchabschnitt in das Fixiermittel einsetzbar ist, in eine Schließstellung, in der der Schlauchabschnitt im Fixiermittel fixiert ist, bewegbar ist.

Dadurch wird erreicht, dass der Katheter bzw. der Schlauchabschnitt in das Fixierungsmittel eingesetzt werden kann, nachdem der Katheter gelegt wurde und seine endgültige Position einnimmt. Das Fixiermittel kann an den jeweiligen Schlauchabschnitt angebracht und schließlich am Patienten angenäht werden. Bei einem Austausch des Katheters bzw. eines Schlauchabschnitts kann vorteilhafterweise das Öffnungsteil aus der Schließstellung wieder in die Öffnungsstellung verlagert, so dass der Schlauchabschnitt aus dem Fixiermittel entnommen werden kann.

Das Aufnahmeteil weist wenigstens eine Öse zur Fixierung des Aufnahmeteils am Patienten auf. Mittels der Öse kann das Aufnahmeteil insbesondere an dem Patienten angenäht werden. Vorzugsweise sind zwei oder mehrere Ösen am Aufnahmeteil vorgesehen.

Das Öffnungsteil weist ferner eine Öse auf, die sich in der Schließstellung in axialer Verlängerung neben der Öse des Aufnahmeteils befindet. Die aneinander anliegenden Ösen können dann von beispielsweise einem Faden gemeinsam durchgriffen werden, wobei dieser Faden ebenfalls zum Annähen des Fixiermittels am Patienten dient. Dadurch kann verhindert werden, dass das Öffnungsteil ohne Entfernung des Fadens geöffnet werden kann.

Das Aufnahmeteil kann insbesondere über ein Filmscharnier mit dem Öffnungsteil verbunden sein. Dadurch ist das Öffnungsteil verliersicher am Aufnahmeteil angeordnet, und kann dennoch aus der Öffnungsstellung in die Schließstellung, und zurück, bewegt werden.

Das Aufnahmeteil sieht vorzugsweise einen Rastabschnitt vor. Das Öffnungsteil sieht vorzugsweise einen mit dem Rastabschnitt zusammenwirkenden Gegenrastabschnitt vor, wobei der Rastabschnitt in der Schließstellung mit dem Gegenrastabschnitt verrastet ist. Das Vorsehen einer derartigen Verrastung hat den Vorteil, dass keine weiteren Bauteile zur Sicherung des Öffnungsteils in der Schließstellung am Aufnahmeteil erforderlich sind.

Die Verrastung ist dabei vorzugsweise derart ausgebildet, dass sie mit einem Werkzeug, und insbesondere mit einem chirurgischen Instrument wie beispielsweise einem Fadenziehmesser lösbar ist. Vorzugsweise ist die Verrastung so, dass sie ohne geeignetes Werkzeug nicht lösbar ist. Dadurch wird erreicht, dass der Patient das Öffnungsteil nicht aus der Schließstellung verlagern kann.

Ferner ist vorteilhaft, wenn das Aufnahmeteil und das Öffnungsteil eine Innenkontur aufweisen, die wenigstens weitgehend komplementär zur Außenkontur des Schlauchabschnitts ausgebildet ist. Hierdurch wird gewährleistet, dass der Schlauchabschnitt sicher im Fixiermittel angeordnet ist. Die Ausbildung kann so sein, dass ein axiales Verschieben des Schlauchabschnitts im Fixiermittel ausgeschlossen wird. Die Ausbildung kann auch so sein, dass zwei miteinander gefügte, in axialer Richtung hintereinander angeordnete Schlauchabschnitte vom Fixiermittel in der Schießstellung sicher miteinander verbunden sind.

Das Aufnahmeteil kann ferner auf seiner dem Patienten zugewandten Unterseite einen flachen Auflageabschnitt aufweisen. Hierdurch wird erreicht, dass das Aufnahmeteil angenehm am Patienten zum Aufliegen kommen kann.

Ferner ist vorteilhaft, wenn der Katheter zwei parallel zueinander verlaufende, jeweils wenigstens ein Lumen bildende Schlauchabschnitte aufweist, wobei ein erstes Fixiermittel vorgesehen ist, das diese beiden Schlauchabschnitte aufnimmt. In einem Schlauchabschnitt kann beispielsweise das dem Patienten entnommene Blut geführt werden; im anderen Schlauchabschnitt das gereinigte, dem Patienten zuzuführende Blut. Proximal zum ersten Fixiermittel ist vorzugsweise ein diese beiden Lumen bildender Schlauchabschnitt vorgesehen, wobei ein zweites Fixiermittel vorgesehen sein kann, das diesen einen Schlauchabschnitt aufnimmt. Über ein entsprechendes Kopplungsmittel können folglich die beiden Lumen, die im distalen Bereich in getrennten Schläuchen laufen, in einen gemeinsamen Schlauchabschnitt überführt werden. Dieser gemeinsame Schlauchabschnitt kann dann ebenfalls mit einem entsprechenden Fixiermittel am Patienten befestigt werden.

Weitere Einzelheiten und vorteilhafte Ausgestaltungen der Erfindung sind der nachfolgenden Beschreibung zu entnehmen, anhand derer Ausführungsbeispiele der Erfindung näher beschrieben und erläutert sind.

Es zeigen:
- Figur 1: das distale Ende eines erfindungsgemäßen Katheters mit zwei Fixiermitteln;
- Figur 2a bis d: ein erstes, in Figur 1 gezeigtes Fixiermittel;
und
- Figur 3a bis d: ein zweites, in Figur 1 gezeigtes Fixiermittel.

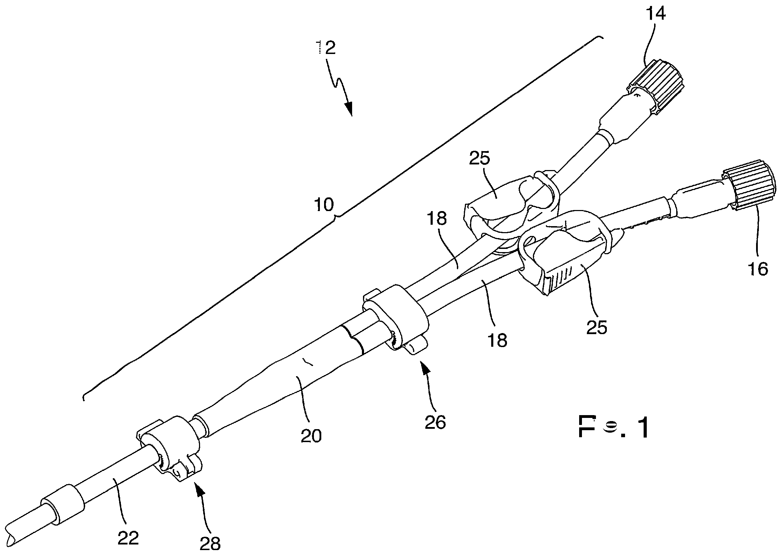

In der Figur 1 ist das distale Ende 10 eines Katheters 12 für die Dialyse gezeigt. Am distalen Ende 10 ist ein Auslass 14 vorgesehen, mit dem entnommenes Blut zu einem Dialysator zugeführt werden kann. Ferner ist ein Einlass 16 vorgesehen, mit dem gereinigtes Blut dem Patienten zugeführt werden kann. Am Auslass 14 sowie am Einlass 16 ist jeweils ein Schlauchabschnitt 18 vorgesehen, wobei die beiden Schlauchabschnitte 18 in ein Kopplungsteil 20 münden. Im Kopplungsteil 20 werden die Lumen der beiden Schlauchabschnitte 18 in einen zweilumigen Schlauchabschnitt 22 eingekoppelt. An den einlumigen Schlauchabschnitten 18 sind jeweils noch Abklemmmittel 25 angeordnet, mit welchen die Schlauchabschnitte 18 verschlossen werden können.

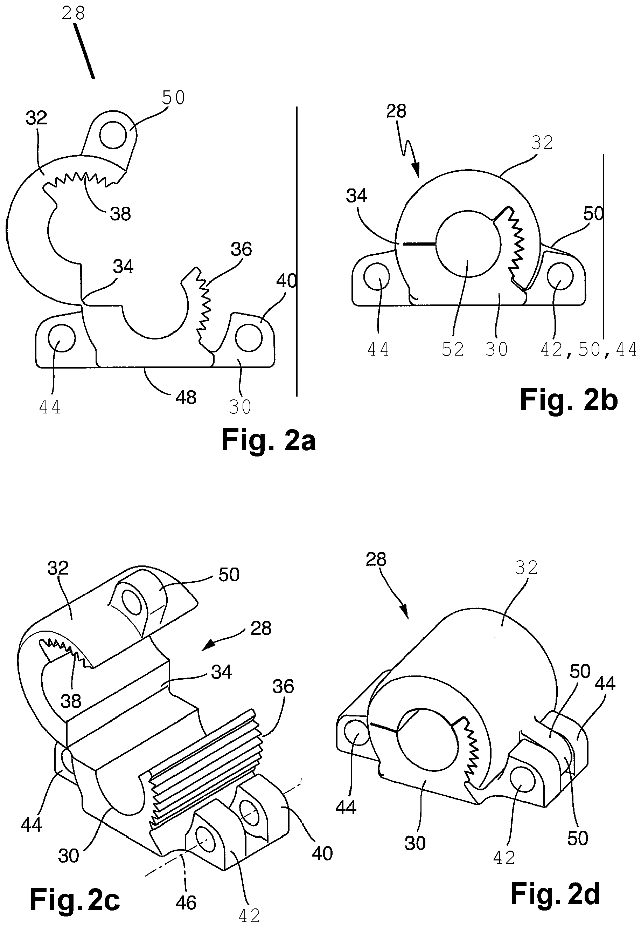

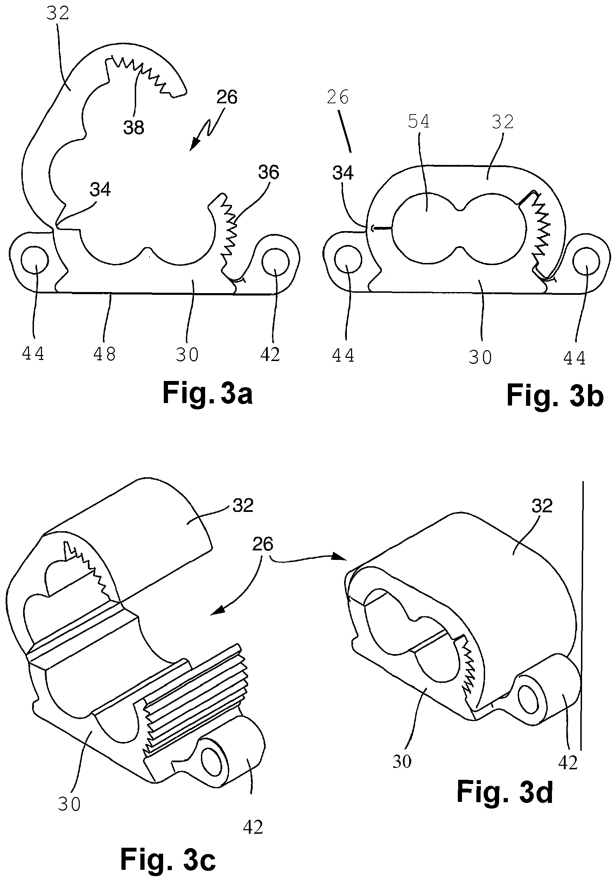

In der Anwendung des Katheters 12 wird das in den Figuren nicht gezeigte proximale Ende in ein Blutgefäß, insbesondere in den rechten Herzvorhof eingeführt. Gerade bei der Langzeitanwendung verbleibt der Katheter 12 mehrere Wochen oder Monate im bzw. am Patienten. Zur sicheren Fixierung des Katheters 12 am Patienten sieht der Katheter 12 zwei Fixiermittel 26 und 28 vor, die in den Figuren 2 und 3 als Einzelteil dargestellt sind. Das Einführen und Legen des Katheters 12 verfolgt dabei ohne die beiden Fixiermittel 26 und 28. Nachdem der Katheter 12 seine endgültige Lage erreicht hat, können die Fixiermittel 26, 28 an den jeweiligen Schlauchabschnitt 18,22 des Katheters 12 angeklipst werden. Das Fixiermittel 26 umschließt dann die beiden einlumigen Schlauchabschnitte 18. Das Fixiermittel 28 umschließt den doppellumigen Schlauchabschnitt 22.

Wie aus den Figuren 2 und 3 deutlich wird, weisen die Fixiermittel 26 und 28 jeweils ein Aufnahmeteil 30 und ein Öffnungsteil 32 auf, wobei das Öffnungsteil 32 aus einer Öffnungsstellung, die in Figur 2a, 2b und 3a, 3b gezeigt ist, in eine Schließstellung, die in Figur 2b, 2d und Figur 3b, 3d gezeigt ist, bewegbar ist. Das Öffnungsteil 32 ist dabei über ein Filmscharnier 34 am Aufnahmeteil 30 angeordnet. Die Fixiermittel 26, 28 sind vorzugsweise aus Kunststoff hergestellt und einstückig ausgebildet.

Die Aufnahmeteile 30 weisen jeweils einen Rastabschnitt 36 auf, der in der Schließstellung mit einem am Öffnungsteil 32 vorgesehenen Gegenrastabschnitt 38 zusammenwirkt. In der Schließstellung liegt der Rastabschnitt 36, bzw. dessen Zähne, im Gegenrastabschnitt 38, bzw. zwischen dessen Zähnen.

Wie aus Figur 2 deutlich wird, sind am Aufnahmeteil 30 insgesamt drei Ösen 40, 42 und 44 angeformt. Die beiden Ösen 40 und 42 sind dabei parallel zueinander verlaufend angeordnet und weisen eine gemeinsame Längsachse 46 auf.

Auf der Unterseite weist das Aufnahmeteil 30 einen flachen Auflageabschnitt 48 auf, der sich bis hin in die Unterseite der Ösen 40, 42 und 44 erstreckt. Der Auflageabschnitt 48 dient zur Anlage an dem Patienten.

Wie ebenfalls aus Figur 2 deutlich wird, ist am Öffnungsteil 32 ebenfalls eine Öse 50 vorgesehen, die in der Schließstellung, wie aus Figur 2a und 2d deutlich wird, zwischen den beiden aufnahmeteilseitigen Ösen 40, 42 zum Liegen kommt. In der Schließstellung liegt folglich die Öse 50 des Öffnungsteils 32 zwischen den beiden aufnahmeteilseitigen Ösen 42, 40.

Das Aufnahmeteil 30 und das Öffnungsteil 32 weisen eine kreisrunde Innenkontur 52 auf, die der Außenkontur des Schlauchabschnitts 22 entspricht, allerdings einen etwas geringeren Durchmesser aufweist, so dass der Schlauchabschnitt 22 in der Schließstellung unter geringer Vorspannung im Fixiermittel angeordnet und in axialer Richtung gesichert ist.

Das Fixiermittel 28 wird am Patienten mittels geeigneten Fäden am Patienten angenäht. Ein Faden wird dabei durch die Ösen 42, 50 und 40 hindurchgeführt und am Patienten befestigt. Ein anderer Faden wird durch die Öse 44 hindurchgeführt und ebenfalls am Patienten vernäht. In der Langzeitanwendung des Katheters 12 kann das Fixiermittel 28, das nahe an der Stelle liegt, an der der Katheter 12 in den Patienten eingeführt wird, nach 4-6 Wochen aus hygienischen Gründen entfernt werden.

Das in der Figur 3 dargestellte Fixiermittel 26 entspricht im Wesentlichen dem Fixiermittel 28. Entsprechende Bauteile sind mit entsprechenden Bezugszeichen versehen. Im Unterschied zum Fixiermittel 28 nimmt das Fixiermittel 26 die beiden parallel zueinander verlaufenden Schlauchabschnitte 18 auf. Deswegen ist die Innenkontur 54 des Fixiermittels 26 entsprechend komplementär zur Außenkontur der Schlauchabschnitte 18 ausgebildet. Im Unterschied zum Fixiermittel 28 kann das Fixiermittel 26 zudem im am Patienten angenähten Zustand geöffnet werden.

Beide Fixiermittel 26 und 28 habe den Vorteil, dass sie erst dann an den jeweiligen Schlauchabschnitten 18, 22 platziert werden können, nachdem der Katheter 12 in den Patienten eingeführt und gelegt wurde.

## Patentansprüche

1. Katheter (12) für die Dialyse mit einem proximalen Ende (10) zum Einführen in ein Blutgefäß, mit einem distalen Ende zur Entnahme von Blut und zur Einführung von gereinigtem Blut, und mit wenigstens einem einen Schlauchabschnitt (18, 22) umgebenden Fixiermittel (26, 28) zur Fixierung des Katheters am Patienten, wobei das Fixiermittel (26, 28) ein Aufnahmeteil (30) und ein Öffnungsteil (32) aufweist, wobei das Öffnungsteil (32) gegenüber dem Aufnahmeteil (30) aus einer Öffnungsstellung, in der der Schlauchabschnitt (18, 22) in das Fixiermittel (26, 28) einsetzbar ist, in eine Schließstellung, in der der Schlauchabschnitt (18, 22) im Fixiermittel (26, 28) fixiert ist, bewegbar ist, **wobei** das
Aufnahmeteil (30) wenigstens eine Öse (40, 42, 44) aufweist und **dadurch gekennzeichnet, dass**
das Öffnungsteil (32) wenigstens eine Öse (50) aufweist, wobei sich die Öse (50) des Öffnungsteils (32) in der Schließstellung in axialer Verlängerung neben der Öse (40, 42, 44) des Aufnahmeteils (30) befindet, so dass die aneinander anliegenden Ösen von einem Faden zur Fixierung des Fixiermittels (26, 28) am Patienten gemeinsam durchgriffen werden können.

2. Katheter (12) nach Anspruch 1, **dadurch gekennzeichnet, dass** das Aufnahmeteil (30) über ein Filmscharnier (34) mit dem Öffnungsteil (32) verbunden ist.

3. Katheter (12) nach Anspruch 1 oder 2, **dadurch gekennzeichnet, dass** das Aufnahmeteil (30) einen Rastabschnitt (36) und dass das Öffnungsteil (32) einen Gegenrastabschnitt (38)aufweist, wobei der Rastabschnitt (36) in der Schließstellung mit dem Gegenrastabschnitt (38) verrastet ist.

4. Katheter (12) nach Anspruch 3, **dadurch gekennzeichnet, dass** der Rastabschnitt (36) und der Gegenrastabschnitt (38) derart ausgebildet sind, dass der Gegenrastabschnitt (38) aus demRastabschnitt (36) mittels eines Werkzeuges lösbar ist.

5. Katheter (12) nach einem der vorhergehenden Ansprüche, **dadurch gekennzeichnet, dass** das Aufnahmeteil (30) und das Öffnungsteil (32) eine Innenkontur (52, 54) aufweisen, die wenigstens weitgehend komplementär zur Außenkontur des Schlauchabschnitts (18, 22) ausgebildet ist.

6. Katheter (12) nach einem der vorhergehenden Ansprüche, **dadurch gekennzeichnet, dass** das Aufnahmeteil (30) auf seiner dem Patienten zugewandten Unterseite einen flachen Auflageabschnitt (48) aufweist.

7. Katheter (12) nach einem der vorhergehenden Ansprüche, **dadurch gekennzeichnet, dass** der Katheter (12) zwei parallel zueinander verlaufende, jeweils wenigstens ein Lumen bildende Schlauchabschnitte (18) aufweist, wobei ein erstes Fixiermittel (26) vorgesehen ist, das diese beiden Schlauchabschnitte (18) aufnimmt und dass proximal zum ersten Fixiermittel ein zweites Fixiermittel (28) vorgesehen ist, das einen die wenigstens beiden Lumen bildenden Schlauchabschnitt (22) aufnimmt.

## Claims

1. Catheter (12) for dialysis comprising a proximal end (10) for introducing into a blood vessel, a distal end for extracting blood and for introducing purified blood, and at least one fixing means (26, 28) surrounding a tube section (18, 22) for fixing the catheter on the patient, wherein the fixing means (26, 28) has a receiving part (30) and an opening part (32), wherein, with respect to the receiving part (30), the opening part (32) can be moved from an opening position, in which the tube section (18, 22) is insertable into the fixing means (26, 28), into a closing position, in which the tube section (18, 22) is fixed in the fixing means (26, 28), wherein the receiving part (30) has at least one eyelet (40, 42, 44) and **characterized in that** the opening part (32) has at least one eyelet (50), wherein the eyelet (50) of the opening part (32) is located in the closing position in axial extension next to the eyelet (40, 42, 44) of the receiving part (30), such that the adjacent eyelets can be penetrated together by a thread for fixing the fixing means (26, 28) on the patient.

2. Catheter (12) according to claim 1, **characterized in that** the receiving part (30) is connected to the opening part (32) by means of a film hinge (34).

3. Catheter (12) according to claim 1 or 2, **characterized in that** the receiving part (30) has a latching section (36) and that the opening part (32) has a counter-latching section (38), wherein the latching section (36) is locked in the closing position with the counter-latching section (38).

4. Catheter (12) according to claim 3, **characterized in that** the latching section (36) and the counter-latching section (38) are designed such that the counter-latching section (38) can be released from the latching section (36) by means of a tool.

5. Catheter (12) according to any of the preceding claims, **characterized in that** the receiving part (30) and the opening part (32) have an inner contour (52, 54) which is at least largely complementary to the outer contour of the tube section (18, 22).

6. Catheter (12) according to any of the preceding claims, **characterized in that** the receiving part (30) has a flat support section (48) on its underside facing the patient.

7. Catheter (12) according to any of the preceding claims, **characterized in that** the catheter (12) has two tube sections (18) which run parallel to one another and each form at least one lumen, wherein a first fixing means (26) is provided which receives these two tube sections (18) and **in that** a second fixing means (28) is provided proximal to the first fixing means and receives a tube section (22) forming the at least two lumens.

## Revendications

1. Cathéter (12) pour la dialyse avec une extrémité proximale (10) pour l'introduction dans un vaisseau sanguin, avec une extrémité distale pour le prélèvement de sang et pour l'introduction de sang purifié, et avec au moins un moyen de fixation (26, 28) entourant une section de tuyau (18, 22) pour la fixation du cathéter sur le patient, dans lequel le moyen de fixation (26, 28) présente une partie de réception (30) et une partie d'ouverture (32), dans lequel la partie d'ouverture (32) est mobile par rapport à la partie de réception (30) d'une position d'ouverture, dans laquelle la section de tuyau (18, 22) peut être insérée dans le moyen de fixation (26, 28), dans une position de fermeture, dans laquelle la section de tuyau (18, 22) est fixée dans le moyen de fixation (26, 28), dans lequel la partie de réception (30) présente au moins un œillet (40, 42, 44) et **caractérisé en ce que**
la partie d'ouverture (32) présente au moins un œillet (50), dans lequel l'œillet (50) de la partie d'ouverture (32) dans la position de fermeture se trouve dans le prolongement axial à côté de l'œillet (40, 42, 44) de la partie de réception (30), de sorte que les œillets appliqués les uns contre les autres peuvent être traversés conjointement par un fil pour la fixation du moyen de fixation (26, 28) sur le patient.

2. Cathéter (12) selon la revendication 1, **caractérisé en ce que** la partie de réception (30) est reliée à la partie d'ouverture (32) par l'intermédiaire d'une charnière film (34) .

3. Cathéter (12) selon la revendication 1 ou 2, **caractérisé en ce que** la partie de réception (30) présente une section d'encliquetage (36) et que la partie d'ouverture (32) présente une section d'encliquetage complémentaire (38), dans lequel la section d'encliquetage (36) est encliquetée avec la section d'encliquetage complémentaire (38) dans la position de fermeture.

4. Cathéter (12) selon la revendication 3, **caractérisé en ce que** la section d'encliquetage (36) et la section d'encliquetage complémentaire (38) sont réalisées de telle sorte que la section d'encliquetage complémentaire (38) peut être libérée de la section d'encliquetage (36) au moyen d'un outil.

5. Cathéter (12) selon l'une quelconque des revendications précédentes, **caractérisé en ce que** la partie de réception (30) et la partie d'ouverture (32) présentent un contour intérieur (52, 54), qui est réalisé de manière au moins en grande partie complémentaire par rapport au contour extérieur de la section de tuyau (18, 22).

6. Cathéter (12) selon l'une quelconque des revendications précédentes, **caractérisé en ce que** la partie de réception (30) présente sur sa face inférieure tournée vers le patient une section d'appui (48) plate.

7. Cathéter (12) selon l'une quelconque des revendications précédentes, **caractérisé en ce que** le cathéter (12) présente deux sections de tuyau (18) s'étendant parallèlement l'une à l'autre, formant respectivement au moins une lumière, dans lequel un premier moyen de fixation (26) est prévu, qui reçoit ces deux sections de tuyau (18) et qu'un deuxième moyen de fixation (28) est prévu de manière proximale par rapport au premier moyen de fixation, qui reçoit une section de tuyau (22) formant les au moins deux lumières.
